# DEMANDE DE BREVET EUROPEEN

(11) **EP 1 552 809 A1**
(43) Date de publication de la demande: **13.07.2005**
(21) Numéro de dépôt: 04292986.9
(22) Date de dépôt: 14.12.2004
(51) Int. Cl.: A61K 7/075

(54) **Composition cosmétique comprenant un tensioactif cationique, un polymère filant de haut poids moléculaire et un épaississant et traitement cosmétique**

(30) Priorité: 07.01.2004 FR 0450028; 07.01.2004 FR 0450034
(71) Demandeur: L'OREAL, 75008 Paris (FR)
(72) Inventeur: Fack, Géraldine, 92300 Levallois (FR)
(74) Mandataire: Le Blainvaux, Françoise

(57) **Abrégé**

La présente invention est relative à une composition cosmétique comprenant, dans un milieu cosmétiquement acceptable, au moins au moins un tensioactif cationique, au moins un polymère filant et au moins un polymère épaississant non cellulosique et à un procédé de traitement cosmétique des matières kératiniques en particulier les cheveux.

Ces compositions possèdent un effet coiffant amélioré.

## Description

La présente invention est relative à une composition cosmétique notamment de conditionnement des cheveux comprenant un tensioactif cationique, au moins un polymère épaississant non cellulosique et au moins un polymère filant particulier et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques contenant tensioactifs cationiques et des polysaccharides épaississants tels que notamment l'amidon ou les celluloses.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

De plus il est le plus souvent nécessaire d'utiliser un deuxième produit pour donner une forme à la chevelure et fixer la coiffure.

En résumé, il s'avère que les compositions cosmétiques actuelles de conditionnement, ne donnent pas complètement satisfaction. Ainsi, on cherche à obtenir des compositions cosmétiques ayant des propriétés coiffantes rincées équivalentes à celles des gels de fixation ou des laques tout en ayant de bonnes propriétés de conditionnement.

La demanderesse a maintenant découvert que l'association d'un tensioactif cationique, de certains polymères présentant un pouvoir filant particulier et d'un polymère épaississant non cellulosique, notamment dans des milieux non détergents ayant une concentration faible ou nulle en agents tensioactifs lavants permet de remédier à ces inconvénients.

Les cheveux traités avec cette composition sont lisses, se démêlent facilement sont brillants, lisses, souples, individualisés et ont un toucher doux et sans résidus. Les cheveux ont un aspect naturel et non chargé.

De plus, les compositions se rincent facilement.

Ainsi, selon la présente invention, il est maintenant proposé de nouvelles compositions cosmétiques non lavantes , comprenant, dans un milieu aqueux cosmétiquement acceptable, au moins un tensioactif cationique, au moins un polymère épaississant non cellulosique et au moins un polymère présentant un pouvoir filant supérieur à 5 cm.

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme après-shampoing.

D'autres objets, caractéristiques, aspects et avantages de l'invention apparaîtront encore plus clairement à la lecture de la description et des divers exemples qui suivent.

Le pouvoir filant d'un polymère correspond à la longueur du fil de polymère obtenu à la rupture dudit fil selon le mode opératoire défini ci-dessous.

Le pouvoir filant des polymères utilisables selon l'invention est celui qui est mesurée pour une composition contenant (% en poids):

| | |
|---|---|
| Lauryléthersulfate de sodium oxyéthyléné à 2,2 moles d'oxyde d'éthylène | 12,5% |
| Cocoamidopropylbétaïne | 2,5 % |
| Polymère à tester | 1 % |
| Eau qsp | 100%. |

La mesure du pouvoir filant se fait à l'aide d'un analyseur de texture TA-TX2 (Rhéo/stable Micro Systems)

La mesure se fait après compression du produit :
- Déplacement du disque (cylindre 35 mm en aluminium) à la vitesse de 2,5 mm/s et détection de la force de résistance à la compression
- Pénétration dans le produit à la même vitesse sur une profondeur de 10 mm
- Retrait de la sonde à la vitesse de 2.5 mm/s
- Mesure du déplacement de la sonde et détection de la rupture du fil de produit

Selon la présente invention, les polymères présentant un pouvoir filant supérieur à 5 cm seront aussi appelés polymères filants.

Les polymères présentant un pouvoir filant supérieur à 5 cm sont notamment soit (a1) une dispersion de particules d'un polymère cationique hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé, ou soit (a2) une solution aqueuse d'un polymère cationique hydrosoluble ayant une masse moléculaire moyenne en poids supérieure à 10⁶, obtenu par polymérisation radicalaire hétérogène de monomères hydrosolubles avec précipitation du polymère formé.

Par polymère cationique, on comprend les polymères comprenant des monomères cationiques et éventuellement des monomères non-ioniques.

Le polymère cationique hydrosoluble de haut poids moléculaire est un polyélectrolyte polymérisé à partir d'au moins un monomère cationique de formule (I).

Comme indiqué ci-dessus, la synthèse des polymères hydrosolubles de haut poids moléculaire, utilisés dans la présente invention, se fait par polymérisation radicalaire hétérogène de monomères hydrosolubles comportant au moins une insaturation éthylénique. La polymérisation se fait dans une solution aqueuse d'un électrolyte minéral (sel) ayant une force ionique suffisante pour provoquer la précipitation du polymère formé dès que celui-ci atteint une certaine masse moléculaire. Cette technique de polymérisation permet ainsi, grâce au phénomène bien connu de relargage par des sels *(salting-out),* la préparation de dispersions aqueuses salines de particules de polymères hydrosolubles. Les polymères ainsi synthétisés se distinguent par une masse moléculaire moyenne en poids élevée, généralement supérieure à 10⁶.

La technique de polymérisation radicalaire hétérogène en milieu aqueux avec précipitation du polymère formé est décrite par exemple dans le brevet US 4 929 655, dans la demande de brevet EP 0 943 628 ou encore dans la demande internationale WO 02/34796.

Pour garantir la stabilité des dispersions de particules de polymère pendant la synthèse et au cours du stockage, il est généralement indispensable de réaliser la polymérisation en présence d'un agent dispersant. Cet agent dispersant est de préférence un polyélectrolyte qui, contrairement au polymère de haut poids moléculaire utilisé dans l'invention, est soluble dans le milieu aqueux de polymérisation de force ionique élevée.

Ce polyélectrolyte dispersant a de préférence une charge identique à celle du polymère synthétisé, autrement dit pour la synthèse de polyélectrolytes cationiques, on utilise généralement un polyélectrolyte dispersant cationique.

On peut citer à titre d'agents dispersants préférés, les polyélectrolytes cationiques obtenus par polymérisation de 50 à 100 % en moles d'au moins un monomère cationique choisi parmi les sels, de préférence les chlorhydrates ou sulfates, de (méth)acrylate de diméthylaminoéthyle, de N-diméthylaminopropyl(méth)acrylamide ou de di(méth)allylamine, le chlorure de (méth)acryloyloxyéthyltriméthylammonium, le chlorure de (méth)acrylamidopropyltriméthylammonium et le chlorure de diméthyldiallylammonium, et de 50 à 0 % en moles d'acrylamide. On peut également utiliser une polyamine telle qu'une polyalkylèneamine.

L'agent dispersant est utilisé de préférence à raison de 1 à 10 % en poids, rapporté au poids total des monomères à polymériser.

La solution aqueuse saline qui sert de milieu de synthèse et de dispersion au polymère hydrosoluble de haut poids moléculaire est une solution d'un ou de plusieurs sels minéraux choisis de préférence parmi les sels anioniques divalents. On peut citer en tant que sels anioniques par exemple le sulfate d'ammonium, l'hydrogénosulfate d'ammonium, le chlorure d'ammonium, le sulfate de sodium, l'hydrogénosulfate de sodium, le sulfate de magnésium, l'hydrogénosulfate de magnésium, le sulfate d'aluminium et l'hydrogénosulfate d'aluminium. Le sulfate d'ammonium et le sulfate de sodium sont particulièrement préférés.

La concentration de ce(s) sel(s) doit de préférence être suffisante pour induire la précipitation du polymère hydrosoluble formé dans le milieu de polymérisation et peut aller jusqu'à la concentration de saturation de chaque sel. Pour obtenir une telle précipitation, la concentration en sel est de préférence au moins égale à 10 % en poids, de préférence supérieure à 15% en poids et généralement inférieure à 50% en poids par rapport au poids total de la solution ou de la dispersion de polymère. La solution aqueuse saline peut contenir en outre des sels monovalents tels que le chlorure de sodium et le chlorure d'ammonium.

La polymérisation radicalaire hétérogène en milieu aqueux telle que décrite ci-dessus s'accompagne le plus souvent d'une augmentation importante de la viscosité du milieu réactionnel, qui se traduit par des difficultés d'agitation, un défaut d'homogénéité du milieu réactionnel et un élargissement de la granulométrie des particules de polymère formé. Pour empêcher une telle augmentation de la viscosité, il a été proposé, dans la demande de brevet européen EP 0 943 628, d'ajouter au milieu de polymérisation au moins un agent empêchant l'augmentation de la viscosité du milieu réactionnel en cours de polymérisation.

Les polymères hydrosolubles de haut poids moléculaire utilisés dans la présente invention sont de préférence préparés en présence d'un tel agent empêchant l'augmentation de la viscosité.

Ces agents empêchant l'augmentation de la viscosité du milieu réactionnel sont choisis par exemple parmi
(1) les acides polycarboxyliques et leurs sels,
(2) les polyphénols,
(3) les composés cycliques contenant un groupe hydroxy et un groupe carboxy, ou leurs sels,
(4) l'acide gluconique ou ses sels,
(5) les produits réactionnels obtenus par réaction d'une méthoxyhydroquinone et/ou d'un monomère cationique (méth)acrylique avec un composé qui génère des radicaux, sous une atmosphère oxydante,
(6) les produits réactionnels obtenus par réaction d'un polymère cationique (méth)acrylique avec un composé qui génère des radicaux, sous une atmosphère oxydante,
(7) les produits réactionnels obtenus par réaction d'un polymère cationique (méth)acrylique avec un agent oxydant,
et leurs mélanges.

L'addition d'au moins un agent empêchant l'augmentation de la viscosité tel que décrit ci-dessus permet d'effectuer la polymérisation des monomères hydrosolubles décrits ci-dessus avec un agitateur à faible puissance tout en évitant la formation de particules grossières. Les agents empêchant l'augmentation de la viscosité sont de préférence solubles dans le milieu réactionnel aqueux.

Comme exemples de composés (A), on peut citer l'acide oxalique, l'acide adipique, l'acide tartrique, l'acide malique, l'acide phtalique et leurs sels.

Comme exemples de composés (B), on peut notamment citer le résorcinol et le pyrogallol.

Comme exemples de composés (C), on peut citer l'acide m-hydroxybenzoïque, l'acide p-hydroxybenzoïque, l'acide salicylique, l'acide gallique, l'acide tannique et les sels de ces acides.

Comme exemples de composés (D), on peut citer le gluconate de sodium, le gluconate de potassium, le gluconate d'ammonium et différents sels aminés de l'acide gluconique.

Comme exemples de composés (E), on peut citer ceux obtenus par réaction d'un composé qui génère des radicaux, sous un courant de gaz oxygéné, dans une solution contenant de la méthoxyhydroquinone et/ou un monomère cationique (méth)acrylique. Le composé qui génère des radicaux peut être un amorceur couramment utilisé pour la polymérisation radicalaire. On peut citer par exemple les amorceurs azoïques hydrosolubles tels que le chlorhydrate de 2,2'-azo-bis(2-amidinopropane) vendu par exemple sous la dénomination V-50 par la société Wako Chemical Industries, ou le chlorhydrate de 2,2'-azo-bis[2-(2-imidazoline-2-yl)propane] vendu par exemple sous la dénomination commerciale VA-044 par la société Wako Chemical Industries, ou un amorceur du groupe des oxydoréducteurs hydrosolubles tels que l'association persulfate d'ammonium/hydrogénosulfite de sodium.

On peut obtenir un agent empêchant l'augmentation de la viscosité (F) par réaction d'un amorceur radicalaire, sous atmosphère oxygénée, avec un agent dispersant selon l'invention. L'amorceur de polymérisation peut être un amorceur azoïque hydrosoluble ou un oxydoréducteur hydrosoluble tels que décrits ci-dessus.

Les composés (G) peuvent être obtenus sous la forme de polymères oxydés de faible masse moléculaire par oxydation d'un agent dispersant cationique selon l'invention obtenu par polymérisation d'un monomère cationique (méth)acrylique, utilisant en tant qu'agent oxydant du peroxyde d'hydrogène ou un halogène.

Comme monomères cationiques (méth)acryliques utilisés pour la préparation des agents empêchant l'augmentation de la viscosité (E), (F) et (G), on peut citer par exemple le chlorhydrate ou le sulfate de (méth)acrylate de diméthylaminoéthyle, le chlorure de (méth)acryloyloxyéthyltriméthyl-ammonium, le chlorure de (méth)acryloyloxyéthyldiméthylbenzylammonium, le chlorhydrate ou le sulfate dérivé du N-diméthylaminopropyl(meth)-acrylamide, le chlorure de (méth)acrylamidopropyltriméthylammonium, le chlorure ou le sulfate de (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium.

Ces agents empêchant l'augmentation de la viscosité (A) à (G) peuvent être utilisés seuls ou en mélange, en une quantité comprise de préférence entre 10 ppm et 10 000 ppm par rapport au poids total de la solution réactionnelle.

Les monomères hydrosolubles polymérisés par polymérisation radicalaire hétérogène en vue de l'obtention des polymères cationiques hydrosolubles de haut poids moléculaire sont des monomères comportant au moins une double liaison éthylénique, par exemple une double liaison vinylique, acrylique ou allylique. Ils peuvent être cationiques, ou non-ioniques et être utilisés en mélange.

A titre de monomères non-ioniques hydrosolubles, on peut citer l'acrylamide, le méthacrylamide, le N-vinylformamide, le N-vinylacétonamide, l'acrylate d'hydroxypropyle et le méthacrylate d'hydroxypropyle.

Les monomères cationiques hydrosolubles sont de préférence choisis parmi les sels de di(alkyle en C₁₋₄)-diallylammonium et les composés de formule (I) 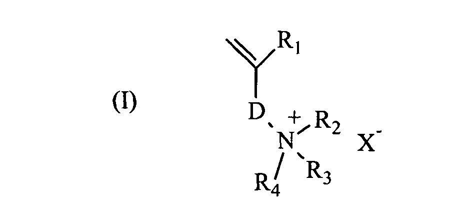 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 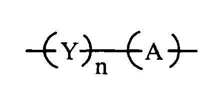 dans lequel Y représente une fonction amide (-CO-NH-), ester (-O-CO- ou -CO-O-), uréthane (-O-CO-NH-) ou urée (-NH-CO-NH-),
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X- représente un contre-ion anionique tel qu'un ion chlorure ou sulfate.

A titre d'exemples de monomères cationiques hydrosolubles, on peut citer le chlorhydrate ou le sulfate de (méth)acrylate de diméthylaminoéthyle, le chlorure de (méth)acryloyloxyéthyltriméthyl-ammonium, le chlorure de (méth)acryloyloxyéthyldiméthylbenzylammonium, le chlorhydrate ou sulfate de N-diméthylaminopropyl-(méth)acrylamide, le chlorure de (méth)acrylamidopropyltriméthylammonium, le chlorure de (méth)acrylamidopropyldiméthylbenzylammonium, le chlorhydrate ou sulfate de (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium et le chlorure de diméthyldiallylammonium.

Dans un mode de réalisation préféré de la présente invention, le polymère hydrosoluble de haut poids moléculaire est obtenu par polymérisation radicalaire hétérogène d'un mélange de monomères comprenant, de 0 à 95,5 % en moles d'acrylamide et de 4,5 à 100 % en moles d'au moins un monomère cationique de formule (I).

Selon un mode de réalisation particulièrement préféré, les polymères hydrosolubles sont obtenus par polymérisation d'un mélange de monomères constitué d'acrylamide et d'un monomère cationique de formule (1), dans lequel le nombre de moles de monomère cationique de formule (I) est supérieur au nombre de moles d'acrylamide.

Des polymères cationiques hydrosolubles particulièrement préférés sont par exemple ceux polymérisés à partir de mélanges de monomères constitués respectivement
1. de 10 % en moles de chlorure d'acryloyloxyéthyldiméthylbenzylammonium et de 90 % en moles d'acrylamide.
2. de 30 % en moles de chlorure d'acryloyloxytriméthylammonium, de 50 % en moles de chlorure d'acryloyloxyéthyldiméthybenzylammonium et de 20 % en moles d'acrylamide.
3. de 10 % en moles de chlorure d'acryloyloxyéthyltriméthylammonium et de 90 % en moles d'acrylamide.
4. de 30 % en moles de chlorure de diallyldiméthylammonium et de 70 % en moles d'acrylamide.

Les polymères hydrosolubles utilisés dans la présente invention ont une masse moléculaire moyenne en poids supérieure à 1 000 000, de préférence allant de 1 000 000 et 50 000 000 et plus particulièrement allant de 1 000 000 et 20 000 000. Cette masse moléculaire moyenne en poids est déterminée par la méthode RSV *(Reduced Specific Viscosity)* telle que définie dans « Principles of Polymer Chemistry », Cornell University Press, Ithaca, NY, 1953, Chapitre VII intitulé « Determination of Molecular Weight », pages 266 - 316.

La concentration de la dispersion ou de la solution de polymère hydrosoluble de haut poids moléculaire est choisie de préférence de manière à ce que la concentration du polymère hydrosoluble aille de 0,01 à 10 % en poids, de préférence de 0,05 à 5 % en poids par rapport au poids total de la composition finale.

La concentration du polymère filant selon l'invention va de préférence de 0,01 à 10 % en poids, de préférence de 0,05 à 5 % en poids par rapport au poids total de la composition finale.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :
- ceux qui présentent la formule générale (V) suivante : 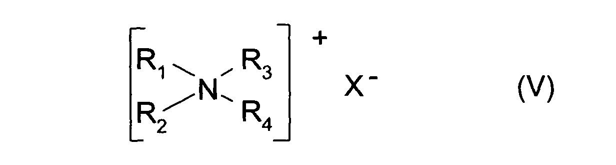
dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (VI) suivante : 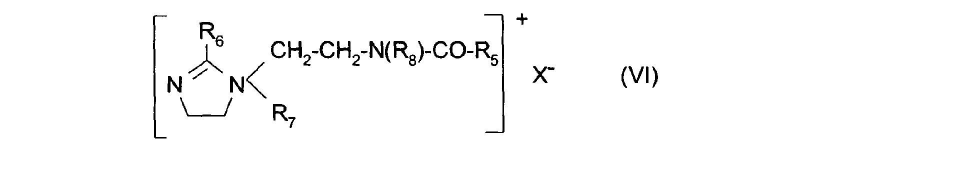
dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
- les sels de diammonium quaternaire de formule (VII) : 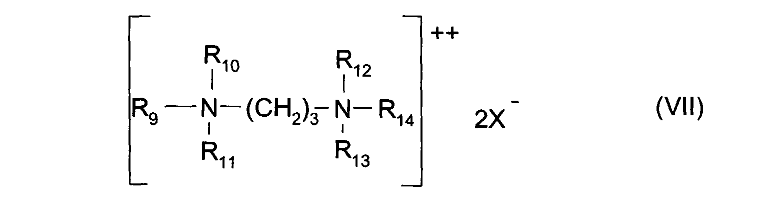
dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire contenant au moins une fonction ester, tels que ceux de formule (VIII) suivante : 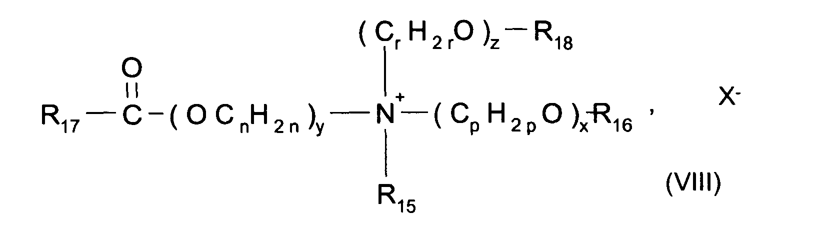
dans laquelle :
R₁₅ est choisi parmi les radicaux alkyles en C₁-C₆ et les radicaux hydroxyalkyles ou dihydroxyalkyles en C₁-C₆ ;
R₁₆ est choisi parmi :
   - le radical 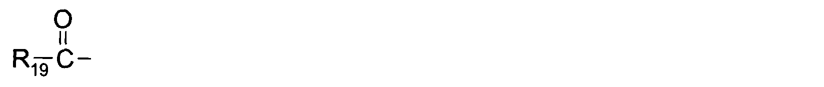
   - les radicaux R₂₀ hydrocarbonés en C₁-C₂₂, linéaires ou ramifiés, saturés ou insaturés,
   - l'atome d'hydrogène,
R₁₇ est choisi parmi :
   - le radical 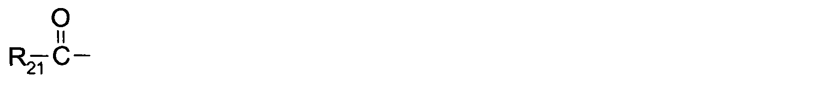
   - les radicaux R₂₂ hydrocarbonés en C₁-C₆, linéaires ou ramifiés, saturés ou insaturés,
   - l'atome d'hydrogène,
R₁₇, R₁₉ et R₂₁, identiques ou différents, sont choisis parmi les radicaux hydrocarbonés en C₇-C₂₁, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10;
X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés, et plus particulièrement linéaires.

De préférence, R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou
différents, valent 0 ou 1.

Avantageusement, y est égal à 1.

De préférence, r, n et p, identiques ou différents, valent 2 ou 3, et encore plus particulièrement sont égaux à 2.

L'anion X- est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, n et pt sont égaux à 2 ;
- R₁₆ est choisi parmi :
- le radical 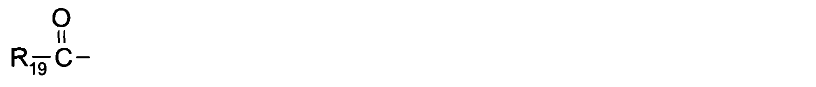
- les radicaux méthyle, éthyle ou hydrocarbonés en C₁₄-C₂₂,
- l'atome d'hydrogène ;
- R₁₈ est choisi parmi :
- le radical 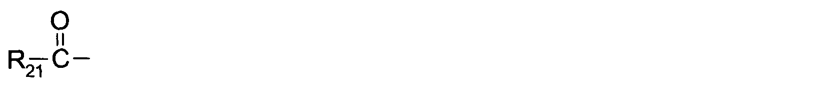
- l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthylméthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyldihydroxyéthyl-méthylammonium, 45 à 60% de méthylsulfate de diacyloxyéthyl-hydroxyéthyl-méthylammonium et 15 à 30% de méthylsulfate de triacyloxyéthylméthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

La composition selon l'invention contient de préférence le ou les tensioactifs cationiques en une quantité allant de 0,05 à 10 % en poids, de préférence de 0,1 à 8 % en poids par rapport au poids total de la composition et plus particulièrement de 0,2 à 5% en poids.

Par l'expression "polymère épaississant non cellulosique", on entend selon l'invention, un polymère épaississant ne comportant pas de motif cellulose.

Les polymères épaississants non cellulosiques sont différents du polymère cationique hydrosoluble de haut poids moléculaire selon l'invention.

Les épaississants non cellulosiques selon l'invention peuvent être d'origine naturelle ou synthétique.

Ils sont notamment choisis parmi :
(i) les homopolymères et copolymères contenant des monomères à insaturation éthylénique de type ester et/ou amide,
(ii) les homo ou copolymères de vinylpyrrolidone,
(iii) les polysaccharides non cellulosiques.

Les agents épaississants purement synthétiques selon l'invention sont avantageusement des polymères ou copolymères d'acide acrylique et/ou méthacrylique, comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol (CARBOPOL 980, 981, 954, 2984, 5984) ou SYNTHALEN ou le polyglycérylméthacrylate commercialisé par la société GUARDIAN sous la dénomination Lubragel ou encore le polyglycérylacrylate commercialisé sous la dénomination Hispagel par la société HISPANO CHIMICA.

On peut encore utiliser comme agent épaississant les polyéthylèneglycols (PEG) et leurs dérivés.

On peut également utiliser avantageusement, en tant qu'agent épaissisant,
- les homopolymères et copolymères de 2-acrylamido-2-méthylpropane sulfonique réticulés,
- les copolymères d'acrylamide et d'acrylate d'ammonium éventuellement réticulés,
- les homopolymères et les copolymères et de chlorure de méthacryloyloxyéthyl triméthylammonium éventuellement réticulés,
- les copolymères d'acrylamide et d'acide 2-acrylamido 2-méthyl propanesulfonique partiellement ou totalement neutralisés éventuellement réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (environ 50/50 en poids) sous forme de dispersion contenant 50 % en poids dudit copolymère dans de l'huile minérale. Cette dispersion est commercialisée sous le nom de "SALCARE SC92" par la Société CIBA.

On peut utiliser un homopolymère réticulé de chlorure de méthacryloyl oxyéthyl triméthylammonium de préférence sous forme de dispersion inverse. Ces dispersions sont notamment commercialisées sous les nom de "SALCARE SC95" et "SALCARE SC96" par la Société CIBA.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM et Haas.

Parmi les homopolymères ou copolymères contenant des monomères à insaturation éthylénique de type ester et/ou amide, on peut citer les polyamides notamment les produits vendus sous les dénominations de : CYANAMER P250 par la société CYTEC (polyacrylamide); les copolymères méthacrylate de méthyle diméthacrylate d'éthylèneglycol ( PMMA MBX-8C par la société US COSMETICS) ; les copolymères méthacrylate de butyle / méthacrylate de méthyle (ACRYLOID B66 par la société RHOM & HAAS), les polyméthacrylate de méthyle (BPA 500 par la société KOBO);

Les homo ou copolymères de vinylpyrrolidone sont notamment choisis parmi les homopolymères de vinylpyrrolidone réticulés tels que le POLYMER ACP-10 commercialisé par ISP;

Les polysaccharides épaississants sont notamment choisis parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les mannanes, les xylanes, les arabanes, les galactanes, les galacturonanes, la chitine, les chitosanes, les glucoronoxylanes, les arabinoxylanes, les xyloglucanes, les glucomannanes, les acides pectiques et les pectines, les arabinogalactanes, les carraghénines, les agars, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar), et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.
On utilisera de préférence, les amidons, les gommes de guar, et leurs dérivés.

Les polysaccharides peuvent être modifiées ou non modifiées.

Les gommes de guar non modifiées sont par exemple les produits vendus sous la dénomination VIDOGUM GH 175 par la société UNIPECTINE, et sous les dénominations MEYPRO-GUAR 50 et JAGUAR C par la société RHODIA CHIMIE.

Les gommes de guar non-ioniques modifiées sont notamment modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHODIA CHIMIE ou sous la dénomination GALACTASOL 4H4FD2 par la société AQUALON.

De préférence, les polymères des compositions cosmétiques conformes à la présente invention présentent avantageusement en solution ou en dispersion, à 1 % de matière active dans l'eau, une viscosité mesurée au moyen du rhéomètre Rhéomat RM 180, à 25 °C, supérieure à 0,1 ps, et plus avantageusement encore supérieure à 0,2 cp, à un taux de cisaillement de 200 s⁻¹.

Selon l'invention, le ou les polymères épaississants peuvent représenter de 0,001 % à 20 % en poids, de préférence de 0,01 % à 15% en poids et plus particulièrement de 0,1 à 10% en poids par rapport au poids total de la composition finale..

La composition selon l'invention peut contenir éventuellement d'autres tensioactifs que les tensioactifs cationiques.

Les tensioactifs sont généralement présents une quantité allant de 0,1% à 10% en poids environ, de préférence de 0,5% à 8% et encore plus préférentiellement de 1% à 5%, par rapport au poids total de la composition.

La composition selon l'invention contient de préférence au moins un tensioactif choisi parmi les tensioactifs non ioniques.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀- C₁₄) amines ou les oxydes de N-acylaminopropylmorpholine. On notera que les alkylpolyglycosides constituent des tensioactifs non-ioniques rentrant particulièrement bien dans le cadre de la présente invention.

La concentration des tensioactifs non ioniques dans les compositions non détergentes peut varier de 0,1 à 10% en poids et de préférence de 0,5 à 8% en poids et plus particulièrement de 1 à 5% en poids par rapport au poids total de la composition.

Les compositions non lavantes (non détergentes) comprennent de préférence moins de 4% en poids de tensioactifs anioniques et plus particulièrement moins de 1% en poids par rapport au poids total de la composition.

La composition selon l'invention peut comprendre en outre au moins un agent conditionneur choisi parmi les silicones, les polymères cationiques, les esters carboxyliques comprenant au moins 12 atomes de carbone, les huiles végétales, les huiles minérales, les huiles de synthèse telles que les poly(alpha-oléfines), et leurs mélanges.

Les silicones utilisables conformément à l'invention peuvent être solubles ou insolubles dans la composition, et elles peuvent être en particulier des polyorganosiloxanes insolubles dans la composition de l'invention. Elles peuvent se présenter sous forme d'huiles, de cires, de résines ou de gommes. Elles peuvent être utilisées pures ou en émulsion, en dispersion ou en microémulsion.

Les organopolysiloxanes sont définis plus en détail dans l'ouvrage de Walter NOLL "Chemistry and Technology of Silicones", (1968) Academic Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 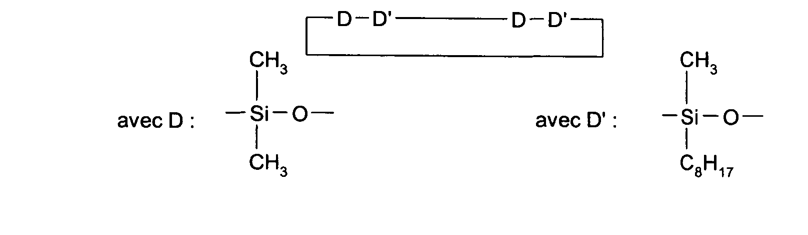
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701^{E} de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255" ;
- des groupements hydroxyacylamino, comme les polyorgano-siloxanes décrits dans la demande EP 342 834. On peut citer, par exemple, le produit Q2-8413 de la société DOW CORNING.

A titre d'exemples de silicones, on utilise de préférence les polydiméthylsiloxanes, les polyalkylarylsiloxanes, les polydiméthylsiloxanes à groupements aminés ou alcoxylés.

La composition selon l'invention peut également comprendre un ou plusieurs esters d'acide carboxylique, tels que par exemple, les composés de formule RₐCOOR_{b} dans laquelle Rₐ représente le reste d'un acide gras supérieur comportant de 4 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle, le néopentanoate d'isostéaryle et leurs mélanges.

La composition selon l'invention peut encore comprendre une ou plusieurs huiles végétales telles que l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum et leurs mélanges.

Comme huiles minérales, on peut notamment citer l'huile de paraffine et l'huile de vaseline.

Les agents conditionneurs sont contenus de préférence dans la composition selon l'invention en une quantité allant de 0,01 % à 20 % en poids, mieux encore allant de 0,1 % à 10 % en poids et plus particulièrement allant de 0,3 % à 5 % en poids par rapport au poids total de la composition.

Le milieu cosmétiquement acceptable est de préférence aqueux et peut être comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀; et leurs mélanges.. Les solvants sont de préférence choisis parmi la glycérine et le propylène glycol.

Le milieu cosmétiquement acceptable notamment aqueux représente de 30 à 98% en poids par rapport au poids total de la composition.

Les solvants sont de préférence présents dans des concentrations allant de 0,5 à 30% en poids par rapport au poids total de la composition.

Le pH des compositions de l'invention va de 2 à 8, de préférence de 3 à 7.

Les compositions selon l'invention peuvent également contenir des additifs classiques bien connus dans la technique, tels que des polymères anioniques, non ioniques ou amphotères, des épaississants non polymériques comme des acides ou des électrolytes, des opacifiants, des nacrants, des vitamines, des provitamines telles que le panthénol, des cires telles que les cires végétales, des céramides naturels ou synthétiques, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux fins. Cet après-shampooing peut être rincé ou non rincé et de préférence rincé.

Les compositions cosmétiques selon l'invention peuvent se présenter sous forme de gel, de lait, de crème, d'émulsion, de lotion fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.

Le rinçage s'effectue par exemple avec de l'eau.

Ainsi, ce procédé selon l'invention permet le maintien de la coiffure, le traitement, le conditionnement, le soin des cheveux ou de toute autre matière kératinique.

Les exemples suivants illustrent la présente invention et ne doivent être considérés en aucune manière comme limitant l'invention.

### EXEMPLES 1 ET 2

On a préparé les compositions suivantes d'après-shampooing à rincer :

| | 1 | 2 |
|---|---|---|
| Copolymère (90/10 en mole) d'acrylamide et de diméthylamino éthyl quaternisé par du chlorure de benzyle (PM>5.10⁶) en dispersion dans une solution aqueuse saline concentrée (ULTIMER de ONDEO) | 1 % MA | 0.5% MA |
| Behenyl trimethyl ammonium chloride | - | 1.5% MA |
| Cetyl trimethyl ammonium chloride | 2.5% MA | - |
| Alcool cétylstéarylique | - | 1.5% MA |
| Amodiméthicone (DC 939 *de DOW CORNING -* 35% *MA)* | 1.5%MA | - |
| Phosphate de di-amidon | 8% | - |
| Polyquaternium- 37 / Mineral oil / PPG1 Trideceth 6 (SALCARE SC95 de CIBA) | - | 1.5% MA |
| NaCl | 2% | - |
| Eau qsp | 100 % | 100 % |

## Revendications

1. Composition cosmétique, comprenant, dans un milieu cosmétiquement acceptable, au moins un tensioactif cationique, au moins un polymère épaississant non cellulosique et au moins un polymère présentant un pouvoir filant supérieur à 5 cm.

2. Composition selon la revendication 1, **caractérisée en ce que** le polymère filant est choisi parmi:
soit (a1) une dispersion de particules d'un polymère cationique hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé, ou soit (a2) une solution aqueuse d'un polymère cationique hydrosoluble ayant une masse moléculaire moyenne en poids supérieure à 10⁶, obtenu par polymérisation radicalaire hétérogène de monomères hydrosolubles avec précipitation du polymère formé.

3. Composition selon la revendication 2, **caractérisé par le fait que** le ou les monomères hydrosolubles sont choisis parmi les monomères cationiques, et/ou non-ioniques comportant au moins une double liaison éthylénique ou un mélange de ceux-ci.

4. Composition selon la revendication 3, **caractérisé par le fait que** les monomères non-ioniques sont choisis parmi l'acrylamide, le méthacrylamide, le N-vinylformamide, le N-vinylacétonamide, l'acrylate d'hydroxypropyle et le méthacrylate d'hydroxypropyle.

5. Composition selon la revendication 3, **caractérisé par le fait que** les monomères cationiques sont choisis parmi les sels de di(alkyle en C₁₋₄)-diallylammonium et les composés de formule (I) 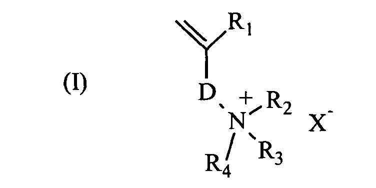 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 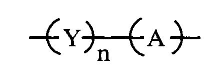 dans lequel Y représente une fonction amide, ester, uréthane ou urée,
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion halogénure ou sulfate.

6. Composition selon l'une quelconque des revendications 2 à 5, **caractérisé par le fait que** le polymère cationique hydrosoluble est polymérisé à partir d'au moins un monomère cationique de formule (I).

7. Composition selon la revendication 6, **caractérisé par le fait que** le polymère cationique hydrosoluble est polymérisé à partir d'un mélange de monomères comprenant de 0 à 95,5 % en moles d'acrylamide et de 4,5 à 100 % en moles d'au moins un monomère cationique de formule (I).

8. Composition selon l'une quelconque des revendications 6 ou 7, **caractérisé par le fait que** le polymère cationique hydrosoluble est polymérisé à partir d'un mélange de monomères constitué d'acrylamide et d'un monomère cationique de formule (I), le nombre de moles du monomère cationique de formule (I) étant supérieur au nombre de moles d'acrylamide.

9. Composition selon l'une quelconque des revendications 6 à 8, **caractérisé par le fait que** le polymère cationique hydrosoluble est polymérisé à partir d'un mélange de monomères constitué de 10 % en moles de chlorure d'acryloyloxyéthyldiméthylbenzyl-ammonium et de 90 % en moles d'acrylamide.

10. Composition selon l'une quelconque des revendications 6 à 8, **caractérisé par le fait que** le polymère cationique hydrosoluble est polymérisé à partir d'un mélange de monomères constitué de 30 % en moles de chlorure d'acryloyloxytriméthylammonium, de 50 % en moles de chlorure d'acryloyloxyéthyldiméthybenzylammonium et de 20 % en moles d'acrylamide.

11. Composition selon l'une quelconque des revendications 6 à 8, **caractérisé par le fait que** le polymère cationique hydrosoluble est polymérisé à partir de 10 % en moles de chlorure d'acryloyloxyéthyltriméthylammonium et de 90 % en moles d'acrylamide.

12. Composition selon l'une quelconque des revendications 6 à 8, **caractérisé par le fait que** le polymère cationique hydrosoluble est polymérisé à partir de 30 % en moles de chlorure de diallyldiméthylammnonium et de 70 % en moles d'acrylamide.

13. Composition selon l'une quelconque des revendications 2 à 12, **caractérisé par le fait que** la concentration des particules de polymère cationique hydrosoluble en dispersion dans une solution aqueuse saline dans (a1) va de 0,01 à 20 % en poids.

14. Composition selon l'une quelconque des revendications 2 à 13, **caractérisé par le fait que** la concentration du polymère hydrosoluble en solution dans (a2) va de 0,01 à 20 % en poids.

15. Composition selon l'une quelconque des revendications 2 à 14, **caractérisé par le fait que** la solution aqueuse saline dans (a1) ou (a2) contient au moins un sel anionique.

16. Composition selon la revendication 15, **caractérisé par le fait que** le sel anionique est choisi parmi le sulfate d'ammonium, l'hydrogénosulfate d'ammonium, le chlorure d'ammonium, le sulfate de sodium, l'hydrogénosulfate de sodium, le sulfate de magnésium, l'hydrogénosulfate de magnésium, le sulfate d'aluminium, l'hydrogénosulfate d'aluminium.

17. Composition selon l'une quelconque des revendications précédentes, **caractérisée par le fait que** les polymères filants sont présents dans les composition de l'invention à une concentration va de 0,01 et 10% en poids, de préférence de 0,05 et 5% par rapport au poids total de la composition.

18. Composition selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

19. Composition selon la revendication 18, **caractérisée en ce que** les sels d'ammonium quaternaire sont choisis parmi :
- ceux qui présentent la formule générale (V) suivante : 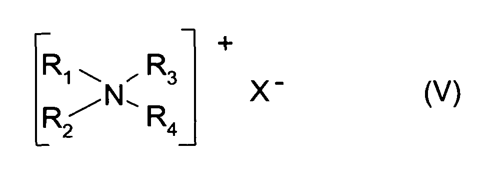
dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle ; X- est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VII) : 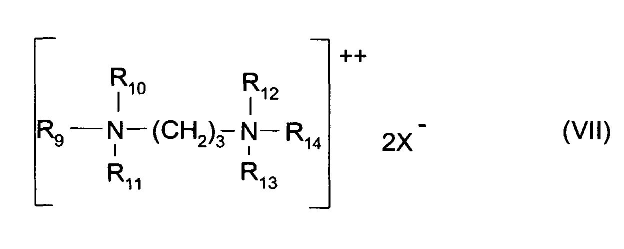
dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates ;
- les sels d'ammonium quaternaire contenant au moins une fonction ester.

20. Composition selon la revendication 19, **caractérisée en ce que** les sels d'ammonium quaternaire de l'imidazoline sont choisis parmi ceux de formule (VI) suivante : 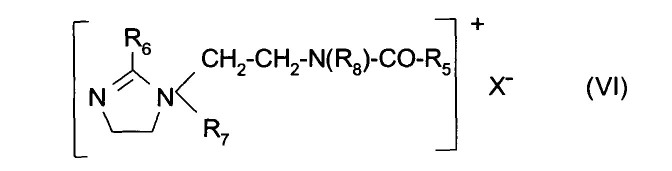 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.

21. Composition selon l'une quelconque des revendications précédentes, **caractérisée en ce que** les tensioactifs cationiques sont choisis parmi le chlorure de béhényltriméthylammonium, le chlorure de cétyltriméthylammonium, le Quaternium-83, le chlorure de béhénylamidopropyl 2,3-dihydroxypropyl diméthyl ammonium et le chlorure de palmitylamidopropyltriméthylammonium.

22. Composition selon l'une quelconque des revendications précédentes, **caractérisée en ce que** les tensioactifs cationiques sont contenus en une quantité de 0,05 à 10 % en poids, de préférence de 0,1 à 8 % en poids par rapport au poids total de la composition.

23. Composition selon l'une quelconque des revendications précédentes, **caractérisée par le fait que** le polymère épaississant non cellulosique est d'origine naturelle ou synthétique.

24. Composition selon la revendication 23, **caractérisée par le fait que** le polymère épaississant est choisis parmi (i) les homopolymères et copolymères contenant des monomères à insaturation éthylénique de type ester et/ou amide,
(ii) les homo ou copolymères de vinylpyrrolidone,
(iii) les polysaccharides non cellulosiques.

25. Composition selon la revendication 23, **caractérisée par le fait que** le polymère épaississant est choisi parmi les polymères ou copolymères d'acide acrylique et/ou méthacrylique.

26. Composition selon la revendication 23 ou 24, **caractérisée par le fait que** le polymère épaississant est un copolymère d'acrylamide ou de dérivé d'acrylamide.

27. Composition selon l'une quelconque des revendications 23 à 26, **caractérisée par le fait que** le polymère épaississant est choisi parmi :
- les homopolymères de 2-acrylamido-2-méthylpropane sulfonique réticulés,
- les copolymères d'acrylamide et d'acrylate d'ammonium éventuellement réticulés,
- les homopolymères et les copolymères de chlorure de méthacryloyloxyéthyl triméthylammonium éventuellement réticulés, et
- les copolymères d'acrylamide et d'acide 2-acrylamido 2-méthyl propanesulfonique partiellement ou totalement neutralisés éventuellement réticulés.

28. Composition selon la revendication 23, **caractérisée par le fait que** le polymère épaississant est choisi parmi les polyéthylèneglycols et leurs dérivés.

29. Composition selon l'une quelconque des revendications 23 ou 24, **caractérisée par le fait que** les polysaccharides sont choisis parmi les glucanes, les amidons modifiés ou non, l'amylose, l'amylopectine, le glycogène, les dextranes, les mannanes, les xylanes, les arabanes, les galactanes, les galacturonanes, la chitine, les chitosanes, les glucoronoxylanes, les arabinoxylanes, les xyloglucanes, les glucomannanes, les acides pectiques et les pectines, les arabinogalactanes, les carraghénines, les agars, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar), et leurs mélanges.

30. Composition selon l'une quelconque des revendications précédentes, **caractérisée en ce que** le polymère épaississant non cellulosique est présent à une concentration allant de 0,001 % à 20 % en poids par rapport au poids total de la composition, de préférence de 0,01 % à 15 % en poids.

31. Composition selon l'une quelconque des revendications précédentes, **caractérisée en ce qu'**elle comprend en outre au moins un agent conditionneur additionnel.

32. Composition selon la revendication 31, **caractérisée en ce que** l'agent conditionneur est choisi parmi les silicones, les polymères cationiques, les esters carboxyliques comprenant au moins 12 atomes de carbone, les huiles végétales, les huiles minérales, les huiles de synthèse et leurs mélanges.

33. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

34. Composition selon la revendication 33, **caractérisée en ce que** le solvant cosmétiquement acceptable est choisi parmi les alcools inférieur en C₁-C₄ tel que l'éthanol, l'isopropanol, le tertio-butanol, le n-butanol ; les alkylèneglycols comme le propylèneglycol, les éthers de polyol ; les alcanes en C₅-C₁₀ et leurs mélanges.

35. Composition selon l'une quelconque des revendications précédentes, **caractérisée en ce qu'**elle comprend en outre des additifs tels que des polymères anioniques, non ioniques ou amphotères, des épaississants comme des acides ou des électrolytes, des opacifiants, des agents nacrants, des vitamines, des provitamines telles que le panthénol, des cires telles que les cires végétales, des céramides naturels ou synthétiques, des parfums, des colorants, des particules organiques ou minérales, des conservateurs, des agents de stabilisation du pH.

36. Composition selon l'une quelconque des revendications précédentes, **caractérisée par le fait qu'**elle se présente sous forme d'après-shampooing, de composition pour la permanente, le défrisage, la coloration ou la décoloration des cheveux, de composition à rincer à appliquer entre les deux étapes d'une permanente ou d'un défrisage.

37. Composition selon l'une quelconque des revendications précédentes, **caractérisée en ce qu'**elle est se présente sous la forme d'un après-shampoing à rincer.

38. Procédé de traitement des matières kératiniques, telles que les cheveux, **caractérisé en ce qu'**il consiste à appliquer sur lesdites matières une quantité efficace d'une composition cosmétique selon l'une des revendications 1 à 37, puis à effectuer éventuellement un rinçage.
